# EUROPÄISCHE PATENTANMELDUNG

(11) **EP 2 921 184 A1**
(43) Veröffentlichungstag der Anmeldung: **23.09.2015**
(21) Anmeldenummer: 14160657.4
(22) Anmeldetag: 19.03.2014
(51) Int. Cl.: A61L 15/34, A61L 15/58, A61K 9/70

(54) **Überpflaster mit verbesserter Verträglichkeit und einer langen Haftungsdauer und Verfahren zu seiner Herstellung**

(71) Anmelder: LTS LOHMANN Therapie-Systeme AG, 56626 Andernach (DE)
(72) Erfinder: Ritzdorf, Gerhard, D-56598 Hammerstein (DE); Hille, Thomas, Dr., D-56567 Neuwied (DE); Botzem, Petra, D-56626 Andernach (DE); Wauer, Gabriel, Dr., D-53474 Ahrweller (DE); Fuhrmann, Marlene, D-56829 Kail (DE)
(74) Vertreter: Schweitzer, Klaus

(57) **Zusammenfassung**

Die Erfindung betrifft ein medizinisches Produkt, bestehend aus einem zentralen haftklebenden Kompartiment (3) und einem wirkstofffreien Überpflaster aus einer wasserdampfdurchlässigen Rückschicht(1) und einer haftklebenden, wirkstofffreien Polymerschicht (2) für eine Fixierdauer von mindesten 7 Tagen mit einer guten Hautverträglichkeit, Verfahren zu Ihrer Herstellung und Laminate aus den Schichten 1 und 2 enthaltende Kits-of-parts.

## Beschreibung

Die vorliegende Erfindung betrifft medizinische Produkte, die auf der Haut getragen werden und durch ein Überpflaster fixiert werden. Dies können transdermale therapeutische Systeme (TTS) sein, die neben dem wirkstoffhaltigen Teil zusätzlich ein wirkstofffreies Überpflaster, welches einen speziellen Aufbau aufweist, aber auch Diagnostika oder Kanülen, die durch eine Pflasterbinde fixiert werden. Bekanntlich sind TTS pflasterförmige Arzneimittel, die auf die Haut aufgeklebt werden und zumindest einen Wirkstoff über die gesamte Applikationsdauer abgeben müssen. Es versteht sich von selbst, dass diese medizinischen Produkte gut kleben müssen, da ansonsten keine Wirkstoffabgabe an die Haut erfolgen kann, das Diagnostikum nicht verlässlich arbeitet oder die Kanüle verrutscht. Gleichzeitig müssen Hautirritationen vermieden werden, das heißt, die Eigenklebrigkeit der TTS, Diagnostikums oder Kanülenfixierpflasters darf nur so hoch wie unbedingt nötig sein.

Bekanntlich versteht man unter Kleben das Verbinden zweier Oberflächen durch einen Klebstoff, der die Oberflächen infolge Adhäsion und Kohäsion miteinander verbindet. Der Klebstoff muss dabei die jeweiligen Oberflächen benetzen. Daraus folgt, dass für gute Klebrigkeit nicht nur der Klebstoff, sondern auch die Beschaffenheit der Oberflächen von Bedeutung sind. Nun haben verschiedene Menschen, besonders mit fortschreitendem Alter Häute mit verschiedenen Oberflächen, wobei die einzelnen Hauttypen mehr oder weniger empfindlich auf Hautreizungen reagieren. Obwohl dieser Bedarf der Fachwelt bekannt ist, gibt es noch keine medizinischen Produkte, die dieser Frage gerecht werden.

Pflaster aus Polyacrylaten über eine Tragedauer bis zu mindestens 7 Tagen genutzt werden können, sind z.B. aus der US 2009/0130190 A1 bekannt. Solche Pflaster führen in der Regel insbesondere bei langer Tragedauer beim Wiederablösen zu merklichen Schmerzen für die betroffene Haut und erhöhten sichtbaren Rückständen an der Haut. Hinzu kommt die gängige Nutzung von Flächengewichten von über 80 g/m² mit dem Nachteil von höheren Restmonomergehalten im gesamten Pflaster. Dies rührt daher, dass bei Überpflastern, die für mindestens 7 Tage ausgelegt sind, oft saure Polyacrylatkleber (Monomere weisen freie Säurefunktionen auf) ohne Zusätze wie Neutralöle genutzt werden, und daher eine sehr starke Bindung zur Haut eingehen.

Als Hautkontaktschichten werden bei wirkstoffhaltigen Silikonklebern oft amin-resistente Silikonkleber genutzt. Diese weisen oft den Nachteil auf, dass diese aufgrund ihrer Amin-Resistenz geringere Bindung zur Haut aufweisen, da alle endständigen Silanolgruppen mit Methylgruppen "gecapped" sind. Daher ist es mit den gängigen Silikonklebern oft sehr schwer eine Tragedauer von mindestens 7 Tagen oder darüber hinaus zu erreichen. Zusätzlich weisen amin-resistente Polysiloxankleber geringere Wasserdampfdurchlässigkeitswerte auf, als dies bei nicht amin-resistenten Versionen der Polysiloxankleber der Fall ist, so dass der Okklusionseffekt bei diesen geringer ausfällt. Bei Nutzung eines Überpflasters, welches mindestens 7 Tage an der Haut mit guter Verträglichkeit haftet, kann dieser Nachteil ausgeglichen werden. Als weiterer Aspekt wird oft als Backing Layer bei wirkstoffhaltigen Silikonpflastern eine einseitig silikonisierte Folie genutzt (z.B. 19 oder 23 µm PET Folie einseitig silikonisiert) Hierbei tritt der nachteilige Effekt auf, dass diese auch auf der nicht-silikonisierten Seite Silikonreste aufweist, da die Rollen in sich gewickelt werden, so dass die nicht silikonisierte Seite in Kontakt zur silikonisierten Seite tritt. Daher ist die Haftung gegenüber der wirkstoffhaltigen Silikonkleberschicht gut, aber gegenüber eines auf Acrylatklebern basierenden Überpflasters geringer. Es kann daher zu verfrühten Ablösungen (bzw. schlechter Haftung) zwischen Überpflaster und wirkstoffhaltiger Matrix mit einer Trennschicht aus einseitig silikonisierter Folie (bzw. Backing Layer) kommen. Mit einem auf nicht amin-resistenten Silikonklebern basierenden wirkstofffreien Überpflasters kann dieser Nachteil ausgeglichen werden.

Aufgabe der vorliegenden Erfindung war daher die Bereitstellung eines wirkstofffreien Überpflasters, welches die Nachteile des Standes der Technik nicht oder nur in weit geringerem Ausmaß aufweist.

Diese Aufgabe wird gelöst durch medizinische Produkte mit einem zentralen Kompartiment und einem wirkstofffreien, das zentrale Kompartiment allseitig überragendem Überpflaster, denn durch diese Konstruktion wird den Anforderungen an standardisierte Produktionsverfahren Rechnung getragen, denn der wesentliche Teil kann in großen Chargen gefertigt werden, während die Varianten, die durch die verschiedenen Hauttypen erforderlich sind, durch unterschiedliche Überpflaster möglich werden. Bevorzugt werden für den klebenden Teil im direkten Hautkontakt Silikonkleber mit erhöhter Wasserdampfdurchlässigkeit und damit verbundenem geringeren Okklusionseffekt oder Polyacrylatkleber mit Zusatz von Neutralöl. Der Silikonkleber kann zudem Silikonöle als Tackifier enthalten. Der Polyacrylatkleber enthält zur besseren Hautverträglichkeit ein Neutralöl, um das Klebeverhalten insbesondere beim Wiederablösen zu verbessern. Als Rückschicht 1 kann ein elastisches, Wasserdampf durchlässiges Gewebe genutzt werden, um dem Pflaster eine gewisse Flexibilität zu geben. Sie ist bevorzugt aus Polyestergewebe und kann zwecks besserer Unterscheidung der Überpflastertypen entsprechend verschieden gefärbt vorliegen. Ein weiterer Bestandteil der Erfindung ist die Nutzung von möglichst geringen Beschichtungsgewichten. Überraschenderweise zeigte sich, dass dadurch die Menge an Restmonomeren, die bei Polyacrylatklebern nach dem Trocknen vorhanden sein können, geringer gehalten werden kann und das Pflaster dennoch 7 Tage haften bleibt. Die Silikonklebervariante zeigt den Vorteil, dass hier überhaupt keine Restmonomere vorhanden sind. Als weitere alternative Polymere für das wirkstofffreie Überpflaster können auch Polyisobutylen/butylen oder Styren-Isopren(alternativ:Butadien)-Styren-Block-Copolymere genutzt werden. Diese können oft zur Erhöhung des Klebeverhaltens mit hydrierten Kohlenwasserstoffharzen und/oder Ölen z.B. Paraffinöl in Kombination eingesetzt werden. Die Harze und Öle werden vorzugsweise in sehr aufgereinigtem Zustand eingesetzt, wodurch die Hautverträglichkeit verbessert ist.

Dieses Überpflaster dient vorzugsweise zur Fixierung von wirkstoffhaltigen Matrixpflastern bestehend aus einer wirkstoffhaltigen Kleberschicht, in der als Kleber amin-resistente Polysiloxankleber, Polyacrylatkleber oder Polyisobutylenkleber Anwendung finden. Diese Kleberschicht ist im direkten Hautkontakt. Als Trennschicht zum wirkstofffreien Überpflaster findet eine Sperrschicht aus PET Folie Anwendung, die bei Silikonklebersystemen meist einseitig silikonisiert ist. Das Überpflaster wird mit überlappenden Rand aufgebracht, so dass der überlappende wirkstofffreie Rand des Überpflasters eine Fixierung über eine Tragedauer von mindestens 7 Tagen gewährleistet.

Die vorliegende Erfindung betrifft daher ein medizinisches Produkt, bestehend aus einem zentralen Kompartiment (3) und einem wirkstofffreien Überpflaster aus
a) einer wasserdampfdurchlässigen Rückschicht(1) und
b) einer haftklebenden, nicht amin-resistente, stark wasserdampfdurchlässige Polysiloxane, Polyacrylate ohne oder nur mit wenig freien Säuregruppen, Polyisobutylene, Polybutylene, Styren-Isopren-Styren-Block-Copolymere oder Styren-Butadien-Styren-Block-Copolymere oder deren Homo- und/oder Copolymere umfassenden wirkstofffreien Polymerschicht (2),
   worin das Kompartiment 3 haftklebend ist und nach Abziehen der wiederablösbaren Schutzschicht (6) direkten Hautkontakt hat,
   das dadurch gekennzeichnet ist, dass die Zusammensetzung der Schicht 2 abhängig von den Bedürfnissen des speziellen Hauttyps des Patienten so gewählt wird, dass das medizinische Produkt für eine Fixierdauer von mindesten 7 Tagen genutzt werden kann (Tragedauer ohne vollständige Ablösung des Pflasters von mindestens 7 Tagen auf menschlicher intakter Haut) und dabei den Anforderungen an eine gute Hautverträglichkeit gerecht wird.
   Vorzugsweise handelt es sich um ein transdermales, therapeutisches System (TTS), worin das Kompartiment 3 aus
c) mindestens einer haftklebenden wirkstoffhaltigen Polymerschicht (5), die nach Abziehen der wiederablösbaren Schutzschicht (6) direkten Hautkontakt hat, und
d) einer die Schicht 5 bedeckenden Trennschicht (4), vorzugsweise aus Polyester, besteht.

Bei dem erfindungsgemäßen medizinischen Produkt kann es sich auch um ein Diagnostikum handeln, bei dem die Diagnosevorrichtung im Kompartiment 3 lokalisiert ist; oder es kann sich um ein Kanülenfixierpflaster handeln, bei dem die Kanüle im Kompartiment 3 lokalisiert ist. Aus der US 2011/144470 A1 sind Diagnosevorrichtungen bekannt, die auf die Haut geklebt werden. Fixiersysteme zur Befestigung von Kanülen auf der Haut sind beispielsweise in der WO 87/07164 A1 beschrieben.

Die bevorzugten TTS sind auf die Haut aufzubringende Arzneiformen mit dem Aussehen traditioneller Pflaster, die über die Haut abzugebende Arzneistoffe enthalten. Ein TTS kann einen oder mehrere Arzneistoffe enthalten, die in vorausbestimmter Rate kontinuierlich über einen festgesetzten Zeitraum an einen festgelegten Anwendungsort abgegeben werden ("Heilmann, Klaus: Therapeutische Systeme Konzept und Realisation programmierter Arzneiverabreichung", 4. Auflage, Ferdinand Enke Verlag Stuttgart, 1984, S. 26).

Die wirkstoffhaltige Haftkleberschicht der TTS besteht aus einer Polymermatrix mit einem Grundpolymer und gegebenenfalls den üblichen Zusätzen. Geeignete Polymere sind Silikone, Kautschuk, kautschukähnliche synthetische Homo-, Co-oder Blockpolymere, Polyacrylsäureester und deren Copolymere und Ester von hydriertem Kolophonium. Grundsätzlich kommen alle Polymere in Frage, die bei der Herstellung von Haftklebern eingesetzt werden und physiologisch unbedenklich sind. Geeignet sind z.B. sind solche, die als Blockcopolymere auf Basis von Styrol und 1,3-Dienen, Polyisobutylenen oder Polymeren und Copolymeren aus Acrylat und/oder Methacrylat bestehen. Von den Blockcopolymeren auf Basis von Styrol und 1,3-Dienen finden z.B. lineare Styrol-Isopren-Styrol Blockcopolymere Verwendung.

Als Polymere auf Acrylatbasis sind beispielsweise Acrylatcopolymere aus 2-Ethylhexylacrylat, Vinylacetat und Acrylsäure mit bzw. ohne Titanchelatester bevorzugt. Als Ester von hydriertem Kolophonium werden vorzugsweise dessen Methyl- und Glycerinester verwendet.

Die Art der als Zusätze möglichen Weichmacher und Klebrigmacher hängt vom verwendeten Polymer ab. Die in Frage kommenden physiologisch unbedenklichen Substanzen sind bekannt. Die Eigenklebrigkeit der Haftkleberschicht muss den dauernden Kontakt zur Haut sicherstellen. Sie kann im Hotmelt-Verfahren, als Lösung oder als Dispersionshaftkleber auf die Rückschicht aufgebracht werden.

Als Wirkstoffe werden Substanzen verwendet, die ohne oder mit Resorptionsvermittler auf der Haut appliziert eine lokale oder systemische Wirkung hervorrufen.

Stoffe mit lokaler Wirkung sind zum Beispiel Antitranspirantia, Fungizide, Bactericide und Bacteriostatica.

Stoffe mit systemischer Wirkung sind beispielsweise Antibiotika, Hormone, Antipyretica, Antidiabetica, Koronardilatatoren, herzwirksame Glycoside, Spasmolytica, Antihypertonica, Psychopharmaka, Migränemittel, Corticoide, Analgetica, Antikontrazeptiva, Antirheumatica, Anticholinergica, Sympatolytica, Sympatomimetica, Vasodilatatoren, Anficoaguiantien und Antiarrhythmika.

Von dem verwandten Polymer und dem Wirkstoff abhängige mögliche Zusätze sind Weichmacher, Klebrigmacher, Stabilisatoren, Trägerstoffe, diffusions- und penetrationsregulierende Zusätze oder Füllstoffe. Die in Frage kommenden physiologisch unbedenklichen Substanzen sind bekannt.

Die Schicht 2 kann 0 bis 5 % (g/g) eines Neutralöls, bezogen auf das trockene Gewicht dieser Schicht, als Verträglichmacher enthalten. Schicht 2 kann auch 0,5 bis 5 % (g/g) Dexpanthenol, bezogen auf das trockene Gewicht dieser Schicht, enthalten.

Schicht 2 kann auch ein nicht amin-resistente Polysiloxanpolymere umfassen, bei welchen nach der Polykondensation des Harz-Anteils und der Polydimethylsiloxanolgruppen die noch freien restlichen Silanolgruppen nicht mit Methylgruppen "gecapped" wurden, dem 0 bis 4 % (g/g) Silikonöl, bezogen auf das trockene Gewicht dieser Schicht, zugesetzt werden.

Schicht 2 kann weiterhin Polyacrylate oder Vinyl-Acrylat-Copolymere umfassen, welche eine Säurezahl von kleiner 1 aufweisen.

Schicht 2 kann schließlich auch Styren-Isopren-Styren-Block-Copolymere oder Styren-Butadien-Styren-Block-Copolymere in Kombination mit hydrierten Kohlenwasserstoffharzen und/oder Ölen wie Paraffinöl umfassen.

Insbesondere weist das bevorzugte TTS die genannten Schichten jeweils einmal auf, und es besteht bevorzugt aus den genannten Schichten. Die beiden genannten haftklebenden Schichten können bezüglich ihrer Polymerzusammensetzung und/oder Dicke gleich oder verschieden sein. Die auf der Haut aufzubringende haftklebende Polymerschicht ist bevorzugt zusätzlich mit einer Schutzschicht bei Acrylat und Polyisobutylenklebern aus z.B. silikonisiertem Papier oder silikonisierter Folie (z.B. PET-Folie) versehen. Bei Silikonklebern findet eine mit Fluorpolymer beschichtete PET Schutzschicht ihre Anwendung. Die einzelnen Pflaster sind in üblicher Weise verpackt, z.B. in einem Vierrandsiegelbeutel aus Verbundpackstoff aus Polyacrylnitril, Aluminiumfolie und Papier.

Die im Hautkontakt stehende haftklebende und hautfreundliche Polymerschicht 2 enthält bevorzugt nicht amin-resistente Polysiloxane, Polyacrylate oder Polyisobutylene. Bevorzugt enthält oder besteht diese Polymerschicht aus einem oder mehreren Vinylacetat-Acrylatcopolymeren, deren Monomere keine bis wenig freien Säuregruppen aufweisen und daher eine Säurezahl von kleiner als 1 aufweisen. Besonders bevorzugt besteht diese Schicht aus einem nicht amin-resistenten Polysiloxan, dass zur Erhöhung des Tacks kleine Mengen an Silikonöl enthalten kann.

Es ist ein besonderes Merkmal des erfindungsgemäßen Überpflasters, dass die im Hautkontakt stehende, haftklebende und hautfreundliche Polymerschicht 2 nicht amin-resistente Polysiloxane mit oder ohne Silikonöl zur Erhöhung des Tacks in sehr kleinen Mengen enthalten kann und in einer bevorzugten Ausführungsform auch enthält.

Es ist ein weiteres besonderes Merkmal des erfindungsgemäßen Überpflasters, dass bei Nutzung eines Polyacrylates dieses Polyacrylat aus der Gruppe der Vinylacetat-Acrylatcopolymere besteht und nach Polymerisation keine bis wenig freie Säuregruppen vorhanden sind und zusätzlich ein Neutralöl für eine Erhöhung der Hautverträglichkeit insbesondere beim Wiederablösen genutzt wird.

Die undurchlässige Trennschicht (4) zwischen wirkstoffhaltiger Schicht und Überpflaster kann aus verschiedenen Polymeren bestehen. Bevorzugt sind hier Polyester und von diesen insbesondere Polyethylenterephthalat. Die Schicht 4 findet Anwendung, sobald eine wirkstoffabgebende Einheit von der wirkstofffreien Überpflastereinheit getrennt werden soll.

Gut haftende Polymere sind beispielsweise Polyacrylate. Besonders gut kleben auf der Haut solche Polyacrylate, die durch Verwendung von freier Acrylsäure hergestellt wurden. Jedoch führen diese zu Hautreizungen und merklichen Schmerzen beim Wiederablösen. In Trageversuchen wurden Polyacrylate genutzt ohne freie Acrylsäure, um eine Tragedauer bei gleichzeitig guter Hautverträglichkeit nachzuweisen. Diese Versuche im Rahmen von 2²-faktor Versuchsplänen waren erfolgreich. Daher können bevorzugt Polymere mit Säurezahlen von < 1 genutzt werden.

Die gleichen Ausführungen gelten grundsätzlich auch für Polysiloxane, wobei bei diesen Polymeren saure Monomere aufgrund ihres anderen chemischen Aufbaus nicht vorkommen. Sie haften daher nur dann zuverlässig über eine Dauer von mindestens 7 Tagen, wenn sie mit nicht amin-resistenten Polysiloxanklebern "Standard PSA" hergestellt werden, um genügend Hydrophile Strukturen mit erhöhter Bindungsaffinität zur Haut aufzuweisen.

Dexpanthenol, das bei dem Überpflaster ebenfalls angewandt werden kann, wird im Körper zu Pantothensäure umgewandelt. Pantothensäure ist ein Vitamin aus der Gruppe der B-Vitamine (Vitamin B5). Pantothensäure ist ein Bestandteil des Coenzyms A und spielt damit eine wesentliche Rolle im Hautstoffwechsel. Aufgrund seiner Hydrophilie findet Dexpanthenol im Rahmen dieser Erfindung bei den unpolaren Polymeren wie Polyisobutylene und Polysiloxane keine Anwendung, da eine Einarbeitung mittels eines hydrophilen Lösemittels erfolgen müsste z.B. Ethanol oder andere Alkohole (Dexpanthenol ist praktisch unlöslich in sehr unpolaren Lösemitteln, wie sie bei Polyisobutylen/Polybutylen und Polysiloxanklebern Anwendung finden z.B. n-Heptan). Die Herstellung würde eine Art Emulsion erfordern mit bekannten Nachteilen wie Phasentrennung, Koaleszenz etc. Bei der Anwendung in Polyacrylaten war eine Einarbeitung von Dexpanthenol einfach, da diese in polaren Lösemitteln wie Gemischen aus Ethylacetat und Ethanol Anwendung finden. Eine Verwendung bei Polyacrylaten führte bei höheren Konzentrationen zu deutlichen Rückständen auf der Haut nach dem Tragen, da es die Kohäsion senkte. Zusätzlich senkt es bei Konzentrationen von z.B. 5 % [w/w] die Tragedauer auf unter 7 Tage aufgrund seiner Weichmachereigenschaften.

Die Erfindung betrifft auch Verfahren zur Herstellung des erfindungsgemäßen medizinisches Produkts, das dadurch gekennzeichnet ist, dass man
- das Kompartiment 3 in an sich bekannter Weise herstellt und dessen hautseitige Klebefläche mit einer allseitig um mindestens 4 mm überstehenden wiederablösbaren Schutzschicht (6) abdeckt,
- den Hauttyp des Patienten beispielsweise nach W. E. Roberts klassifiziert und mit Hilfe dieser Klassifikation dann aus nicht amin-resistenten, stark wasserdampfdurchlässigen Polysiloxane, Polyacrylate ohne oder nur mit wenig freien Säuregruppen, Polyisobutylene, Polybutylene, Styren-Isopren-Styren-Block-Copolymeren oder Styren-Butadien-Styren-Block-Copolymeren oder deren Homo- und/oder Copolymeren umfassenden Gruppe diejenigen Polymere auswählt und gegebenenfalls aus den in den vorangehenden Ansprüchen beanspruchten Zusätzen diejenigen Zusätze auswählt, die erforderlich sind, damit das medizinische Produkt für eine Fixierdauer von mindesten 7 Tagen genutzt werden kann und dabei den Anforderungen an eine gute Hautverträglichkeit gerecht wird,
- daraus Beschichtungsmasse herstellt und diese auf einen Zwischenträger beschichtet und trocknet,
- die Rückschicht 1 auf das so erhaltene Laminat aus Schicht 2 und Zwischenträger auflaminiert und daraus ein Überpflaster einer solchen Größe und Form ausstanzt, welches das Kompartiment 3 allseitig um mindestens 4 mm überragt, und
- das so erhaltene Überpflaster schließlich nach Abziehen des Zwischenträgers auf die von der hautseitigen Klebefläche abgewandte Seite des Kompartiments 3 so aufklebt, dass sie dieses das Kompartiment allseitig um mindestens 4 mm überragt.

Das W.E. Robert Skin Type Classification System wurde in jüngerer Zeit eingeführt, um eine Einteilung des Hauttyps z.B. bei kosmetischen Behandlungen zu ermöglichen (siehe z.B. Roberts, J. Drugs Dermatol. 2008 May; 7(5): 452 - 456 und Robert, Dermatol. Clin. 2009 Oct; 27(4): 529 - 533). Es handelt sich dabei um ein vierteiliges System, das nicht nur die Eigenschaften des Hauttyps bestimmt, sondern auch Informationen zur Prognose der Hautreaktionen liefert.

Die Erfindung betrifft auch ein Kit-of-parts, umfassend
- ein auf der hautseitigen Klebefläche mit einer wiederablösbaren Schutzschicht (6) versehenes Kompartiment 3, das wie in einem der Ansprüche 1 bis 4 definiert ist, und
- mindestens ein auf einen Zwischenträger auflaminiertes wirkstofffreies Überpflaster aus
   a) einer wasserdampfdurchlässigen Rückschicht(1) und
   b) einer haftklebenden, nicht amin-resistente, stark wasserdampfdurchlässige Polysiloxane, Polyacrylate ohne oder nur mit wenig freien Säuregruppen, Polyisobutylene, Polybutylene, Styren-Isopren-Styren-Block-Copolymere oder Styren-Butadien-Styren-Block-Copolymere oder deren Homo- und/oder Copolymere umfassenden wirkstofffreien Polymerschicht (2). Vorzugsweise umfasst das Kit mindestens zwei auf einen Zwischenträger auflaminierte, wie definierte wirkstofffreie Überpflaster, die sich hinsichtlich der Zusammensetzung der Schicht 2, ihrer Größe und/oder ihrer Form unterscheiden. Mit dieser bevorzugten Ausführungsform erhält der Anwender eine "Baukasten", der ihm erlaubt das in einem standardisierten Verfahren gefertigte und strenge pharmazeutische Auflagen erfüllende Kompartiment 3 mit einem geeigneten Überpflaster zu kombinieren, damit das medizinische Produkt den Anforderungen an eine Fixierdauer von mindesten 7 Tagen genutzt werden kann und dabei den Anforderungen an eine gute Hautverträglichkeit gerecht wird.

Die Erfindung betrifft weiterhin ein Kit-of-parts, umfassend
- mindestens zwei auf einen Zwischenträger auflaminierte wirkstofffreie Überpflaster aus
   a) einer wasserdampfdurchlässigen Rückschicht(1) und
   b) einer haftklebenden, nicht amin-resistente, stark wasserdampfdurchlässige Polysiloxane, Polyacrylate ohne oder nur mit wenig freien Säuregruppen, Polyisobutylene, Polybutylene, Styren-Isopren-Styren-Block-Copolymere oder Styren-Butadien-Styren-Block-Copolymere oder deren Homo- und/oder Copolymere umfassenden wirkstofffreien Polymerschicht (2)
wobei sich die Überpflaster hinsichtlich der Zusammensetzung der Schicht 2, ihrer Größe und/oder ihrer Form unterscheiden.

Auch mit diesem Kit erhält der Anwender eine "Baukasten", der ihm erlaubt das in einem standardisierten Verfahren gefertigte und strenge pharmazeutische Auflagen erfüllende Kompartiment 3 mit einem geeigneten Überpflaster zu kombinieren, damit das medizinische Produkt den Anforderungen an eine Fixierdauer von mindesten 7 Tagen genutzt werden kann und dabei den Anforderungen an eine gute Hautverträglichkeit gerecht wird.

Die Erfindung betrifft schließlich die Verwendung eines vorstehend beschriebenen Kit-of-parts bei einem Verfahren zur Herstellung eines medizinisches Produkts, bestehend aus einem zentralen Kompartiment (3) und einem wirkstofffreien Überpflaster aus
a) einer wasserdampfdurchlässigen Rückschicht(1) und
b) einer haftklebenden, nicht amin-resistente, stark wasserdampfdurchlässige Polysiloxane, Polyacrylate ohne oder nur mit wenig freien Säuregruppen, Polyisobutylene, Polybutylene, Styren-Isopren-Styren-Block-Copolymere oder Styren-Butadien-Styren-Block-Copolymere oder deren Homo- und/oder Copolymere umfassenden wirkstofffreien Polymerschicht (2),
worin das Kompartiment 3 haftklebend ist und nach Abziehen der wiederablösbaren Schutzschicht (6) direkten Hautkontakt hat, bei welchem ein auf einen Zwischenträger auflaminiertes wirkstofffreies Überpflaster nach Abziehen des Zwischenträgers auf ein Kompartiment 3 aufgeklebt wird.

Die nachfolgenden besonders bevorzugten Ausführungsformen der vorliegenden Erfindung, die Zeichnungen und die Ausführungsbeispiele dienen zur näheren Erläuterung der Erfindung, ohne diese darauf beschränkt wäre. Vielmehr ist jede für den Fachmann sinnvolle Kombination von (einzelnen) Merkmalen, unabhängig davon, ob sie in der Beschreibung, den Beispielen oder Ansprüchen genannt sind, von der vorliegenden Erfindung umfasst.

In der Zeichnung zeigt:
Fig. 1 einen Schnitt durch das erfindungsgemäße medizinische Produkt.
Fig. 2 einen Schnitt durch das erfindungsgemäße bevorzugte TTS.

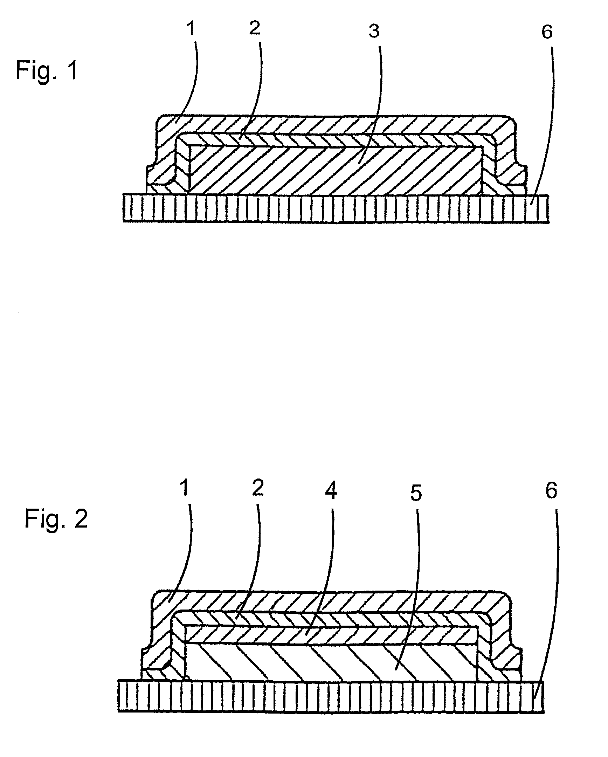

In Fig. 1 und 2 sind die Schichtdicken der Deutlichkeit wegen übertrieben dargestellt. In Fig. 1 ist das haftklebende zentrale Kompartiment (3) schematisch mit einem rechteckigen Querschnitt dargestellt. Der tatsächliche Querschnitt kann davon abweichen.

Wie in Fig. 1 zu sehen ist, ruht Kompartiment 3 auf der wiederablösbaren Schutzschicht (6) und wird von dem aus der wasserdampfdurchlässigen Rückschicht (1) und der haftklebenden wirkstofffreien Polymerschicht (2) gebildeten Überpflaster bedeckt. Es ist zu erkennen, dass Kompartiment 3 von der Rückschicht 1 und der Polymerschicht 2 allseitig überragt wird.

Wie in Fig. 2 zu sehen ist, umfasst das TTS eine haftklebende wirkstoffhaltige Polymerschicht (5), die auf der wiederablösbaren Schutzschicht (6) ruht und von dem aus der wasserdampfdurchlässigen Rückschicht (1) und der haftklebenden wirkstofffreien Polymerschicht (2) gebildeten Überpflaster bedeckt wird. Die Trennschicht (4) trennt dabei die wirkstoffhaltige Schicht 5 von der haftklebenden wirkstofffreien Polymerschicht (2). Es ist zu erkennen, dass aus den Schichten 4 und 5 gebildetes Laminat von der Rückschicht 1 und der Polymerschicht 2 allseitig überragt wird.

In bevorzugten Ausführungsformen umfasst die gut hautverträgliche wirkstofffreie Polymerschicht (2) (hier und im Folgenden angegebene prozentuale Gehaltsgrößen sind Massenanteile [w/w]):
- einem nicht amin-resistenten Polysiloxankleber "Standard PSA" mit einem optionalen Silikonölanteil (z.B. Silikonöl 12500 cST) zur Erhöhung des Tacks von < 4 % und einem Flächengewicht von < 80g/m²;
- einen Polyacrylatkleber ohne freie Säuregruppen mit einem Neutralölanteil (z.B. Miglyol) von < 5 % und einem Flächengewicht von < 80g/m²;
- einen Polyacrylatkleber ohne freie Säuregruppen mit einem Dexpanthenolanteil von < 5 % und einem Flächengewicht von < 80g/m²;
- einen Polyisobutylen/Polybutylenkleber optional mit einem Neutralöl- oder Silikonölanteil (z.B. Miglyol oder Silikonöl 12500 cST) von < 5 % und einem Flächengewicht von < 80g/m²; oder
- einen auf Styren-Isopren(alternativ: Butadien)-Styren (SIS) Block Copolymer basierendem Kleber (Diese werden oft zur Erhöhung des Klebeverhaltens mit hydrierten Kohlenwasserstoffharzen und/oder Ölen wie Paraffinöl in Kombination eingesetzt. Der Styren-Isopren(alternativ zu Isopren: Butadien)-Styren Block Copolymer Anteil kann zwischen 15 - 100 %, der Harzanteil bis zu 70 %, der Ölanteil bis zu 50 % betragen. Das Flächengewicht beträgt < 90g/m².)

In besonders bevorzugten Ausführungsformen umfasst die gut hautverträgliche wirkstofffreie Polymerschicht (2):
- einen nicht amin-resistenten Polysiloxankleber "Standard PSA" mit einem optionalen Silikonölanteil (z.B. Silikonöl 12500 cST) zur Erhöhung des Tacks von ≤ 1 % und einem Flächengewicht von ≤ 70g/m²;
- einen Polyacrylatkleber ohne freie Säuregruppen mit einem Neutralölanteil (z.B. Miglyol) von ≤ 2 % und einem Flächengewicht von < 75g/m²;
- einen Polyacrylatkleber ohne freie Säuregruppen mit einem Dexpanthenolanteil von ≤ 2,5 % und einem Flächengewicht von < 75g/m²;
- einen Polyisobutylen/butylenkleber optional mit einem Neutralöl- oder Silikonölanteil (z.B. Miglyol oder Silikonöl) von ≤ 2 % und einem Flächengewicht von < 70g/m²; oder
- einen auf Styren-Isopren(alternativ zu Isopren: Butadien)-Styren Block Copolymer basierendem Kleber. (Diese werden oft zur Erhöhung des Klebeverhaltens mit hydrierten Kohlenwasserstoffharzen und/oder Ölen wie Paraffinöl in Kombination eingesetzt. Der Styren-Isopren(alternativ zu Isopren: Butadien)-Styren Block Copolymer Anteil kann zwischen 25 - 100 %, der Harzanteil bis zu 55 %, der Ölanteil bis zu 38 % betragen. Das Flächengewicht beträgt < 75g/m².)

Die Trennschicht (4) zwischen Wirkstoff abgebender Einheit 5 und dem aus den Schichten 1 und 2 gebildeten Überpflaster besteht bevorzugt aus Polyethylenterephthalat (PET) mit einer Dicke von 19 - 23 µm. Sie kann bei Nutzung von Polysiloxanklebern in den Schichten 2 oder 5 silikonisiert sein.

### Herstellungsbeispiele:

### Beispiele 1 - 5 (DoE1) mit Polyacrylatkleber + Neutralöl:

Herstellung der Masse für das wirkstofffreie Überpflaster mit Polyacrylatkleber + Neutralöl:

Eine Vinylacetat-Acrylat-copolymerisat Lösung in Etylacetat/Ethanol/Heptan mit einem Feststoffanteil von 40 - 43 % [w/w] wird mit einer Lösung von Neutralöl (Miglyol) versetzt, und der Gesamtfeststoffanteil der Lösung mit Ethanol auf 40 % eingestellt.

Dann wird gerührt, bis eine homogene Lösung entsteht.

Diese Kleberlösung (Masse) wird für die Herstellung der wirkstofffreien, der Haut zugewandten Seite (Schicht 2), des Überpflasters genutzt.

### Beschichtung und Trocknung der Masse auf Zwischenträger:

Die Masse wird so auf ein silikonisiertes PE-Papier beschichtet, dass nach Abdampfen der Lösemittel im Trockenschrank oder Trockenkanal bei maximal 100 °C eine Kleberschicht mit einem Flächengewicht von 50 - 110 g/m² entsteht.

Die eingesetzten Zusammensetzungen der Kleberlösungen für das Überpflaster je Formulierung betrugen bei den DoE1 (Design of Experiment) Versuchen:

**Tabelle 1:**

| Beispiel | Vinylacetat-Acrylat-copolymerisat Lösung 42,1 % (z.B. ®DURO-TAK 387-2515)[g] | Neutralöl (®Miglyol 812)[g] | Ethanol[g] | Beschichtungsgewicht nach dem Trocknen bei ca. 10 - 12 Min bei 60°C [g/m²] |
|---|---|---|---|---|
| Beispiel 1 | 181,6 | 1,6 | 11,9 | 81,1 |
| Beispiel 2 | 368,5 | 0,82 | 30,6 | 52,5 |
| Beispiel 3 | 368,5 | 0,82 | 30,6 | 114,4 |
| Beispiel 4 | 352,2 | 7,8 | 30,2 | 49 |
| Beispiel 5 | 352,2 | 7,8 | 30,2 | 114,1 |

### Beispiele 6 - 10 (DoE2) mit Polyacrylatkleber + Dexpanthenol:

### Herstellung der Masse für das wirkstofffreie Überpflaster mit Polyacrylatkleber + Dexpanthenol:

Eine Vinylacetat-Acrylat-copolymerisat Lösung in Ethylacetat/Ethanol/Heptan mit einem Feststoffanteil von 40 - 43 % [w/w] wird mit Dexpanthenol versetzt, und der Gesamtfeststoffanteil der Lösung mit Ethanol auf 40 % eingestellt.

Dann wird gerührt, bis eine homogene Lösung entsteht.

Diese Kleberlösung (Masse) wird für die Herstellung der wirkstofffreien, der Haut zugewandten Seite (Schicht 2), des Überpflasters genutzt.

### Beschichtung und Trocknung der Masse auf Zwischenträger:

Die Masse wird so auf ein silikonisiertes PE-Papier beschichtet, dass nach Abdampfen der Lösemittel im Trockenschrank oder Trockenkanal bei maximal 100 °C eine Kleberschicht mit einem Flächengewicht von 50 - 110 g/m² entsteht.

Die eingesetzten Zusammensetzungen der Kleberlösungen für das Überpflaster je Formulierung betrugen bei den DoE2 (Design of Experiment) Versuchen (Beispiele 6 - 10):

**Tabelle 2:**

| Beispiel | Vinylacetat-Acrylat-copolymerisat Lösung 42,1 % (z.B. ®DURO-TAK 387-2515)[g] | Dexpanthenol [g] | Ethanol [g] | Beschichtungsgewicht nach dem Trocknen bei ca. 10 - 12 Min bei 60°C [g/m²] |
|---|---|---|---|---|
| Beispiel 6 | 180,4 | 2,1 | 12,5 | 80,7 |
| Beispiel 7 | 368,8 | 0,8 | 20,5 | 47,8 |
| Beispiel 8 | 368,8 | 0,8 | 20,5 | 119,2 |
| Beispiel 9 | 352,9 | 7,9 | 30,2 | 50,6 |
| Beispiel 10 | 352,9 | 7,9 | 30,2 | 113,5 |

### Beispiele 11 - 15 (DoE3) und 16 - 20 (DoE4) mit nicht amin-resistentem Polysiloxankleber + Silikonöl:

### Herstellung der Masse für das wirkstofffreie Überpflaster mit nicht amin-resistentem Polysiloxankleber + Silikonöl:

Eine nicht amin-resistente Polysiloxankleber Lösung in Heptan (alternativ Ethylacetat) mit einem Feststoffanteil von 50 - 70 % [w/w] wird mit Silikonöl versetzt, gegebenenfalls mit n-Heptan (bzw. bei in Ethylacetat gelösten nicht amin-resistenten Polysiloxanklebern mit Ethylacetat) auf 60 - 70 % verdünnt und dann gerührt, bis eine homogene Lösung entsteht.

Diese Kleberlösung (Masse) wird für die Herstellung der wirkstofffreien, der Haut zugewandten Seite (Schicht 2), des Überpflasters genutzt.

### Beschichtung und Trocknung der Masse auf Zwischenträger:

Die Masse wird so auf eine mit Fuorpolymer beschichtete PET Folie (Schutzschicht bzw. Zwischenträger), dass nach Abdampfen der Lösemittel im Trockenschrank oder Trockenkanal bei maximal 100 °C eine Kleberschicht mit einem Flächengewicht von 50 - 80 g/m² entsteht.

Die eingesetzten Zusammensetzungen der Kleberlösungen für das Überpflaster je Formulierung betrugen bei den DoE3 (Design of Experiment) Versuchen:

**Tabelle 3:**

| Beispiel | Nicht amin-resistente Polysiloxan Kleber Lösung 60 % (z. B. ®BIO-PSA 7-4501) [g] | Silikonöl [g] | n-Heptan [g] | Beschichtungsgewicht nach dem Trocknen bei ca. 10Min bei 50°C [g/m²] |
|---|---|---|---|---|
| Beispiel 11 | 323,1 | 1,0 | 0,6 | 54,4 |
| Beispiel 12 | 323,1 | 1,0 | 0,6 | 85,9 |
| Beispiel 13 | 310,9 | 7,9 | 5,2 | 51,9 |
| Beispiel 14 | 310,9 | 7,9 | 5,2 | 81,6 |
| Beispiel 15 | 160,5 | 1,0 | 0,7 | 65,9 |

Die eingesetzten Zusammensetzungen der Kleberlösungen für das Überpflaster je Formulierung betrugen bei den DoE4 (Design of Experiment) Versuchen:

**Tabelle 4:**

| Beispiel | Nicht amin-resistente Polysiloxan Kleber Lösung 61 % (z.B. ®BIO-PSA 7-4601) [g] | Silikonöl [g] | n-Heptan [g] | Beschichtungsgewicht nach dem Trocknen bei ca. 10 Min bei 50°C [g/m²] |
|---|---|---|---|---|
| Beispiel 16 | 311,5 | 1,0 | 3,0 | 59,5 |
| Beispiel 17 | 311,5 | 1,0 | 3,0 | 85,6 |
| Beispiel 18 | 306,4 | 7,9 | 10,2 | 59,2 |
| Beispiel 19 | 306,4 | 7,9 | 10,2 | 84,2 |
| Beispiel20 | 167,4 | 1,0 | 3,4 | 72,6 |

Die getrocknete Kleberoberfläche wird mit einer wasserdampfdurchlässigen Rückschicht 1 z.B. Polyestergewebe, elastisch abgedeckt.

Aus den so entstehenden Laminaten bestehend aus Zwischenträger, getrockneter Polymerschicht 2 und Rückschicht 1 werden nun Überpflaster geeigneter Größe ausgestanzt, um diese z.B. bei Anwendung in Kombination mit einer Wirkstoff abgebenden Schicht 5 auf die Trennschicht 4 zu kleben. Die Stanzung des Überpflasterlaminates erfolgt in der Weise, dass alle Schichten außer dem Zwischenträger durchtrennt werden. Die Größe des Überpflasters in Vergleich zur wirkstoffabgebenden Einheit inklusive der Trennschicht bestimmt hierbei maßgeblich die Größe des überlappenden Randbereiches, der die Fixierung in direktem Hautkontakt ermöglicht. Die Breite des überlappenden Randbereiches sollte nicht weniger als 0,4 cm betragen, um eine ausreichende Fixierung über 7 Tage zu gewährleisten.

## Patentansprüche

1. Medizinisches Produkt, bestehend aus einem zentralen Kompartiment (3) und einem wirkstofffreien Überpflaster aus
a) einer wasserdampfdurchlässigen Rückschicht(1) und
b) einer haftklebenden, nicht amin-resistente, stark wasserdampfdurchlässige Polysiloxane, Polyacrylate ohne oder nur mit wenig freien Säuregruppen, Polyisobutylene, Polybutylene, Styren-Isopren-Styren-Block-Copolymere oder Styren-Butadien-Styren-Block-Copolymere oder deren Homo-und/oder Copolymere umfassenden wirkstofffreien Polymerschicht (2),
worin das Kompartiment 3 haftklebend ist und nach Abziehen der wiederablösbaren Schutzschicht (6) direkten Hautkontakt hat,
**dadurch gekennzeichnet, dass** die Zusammensetzung der Schicht 2 abhängig von den Bedürfnissen des speziellen Hauttyps des Patienten so gewählt wird, dass das medizinische Produkt für eine Fixierdauer von mindesten 7 Tagen genutzt werden kann und dabei den Anforderungen an eine gute Hautverträglichkeit gerecht wird.

2. Medizinisches Produkt gemäß Anspruch 1, **dadurch gekennzeichnet, dass** es sich um ein transdermales, therapeutisches System (TTS) handelt, worin das Kompartiment 3 aus
c) mindestens einer haftklebenden wirkstoffhaltigen Polymerschicht (5), die nach Abziehen der wiederablösbaren Schutzschicht (6) direkten Hautkontakt hat, und
d) einer die Schicht 5 bedeckenden Trennschicht (4), vorzugsweise aus Polyester,
besteht.

3. Medizinisches Produkt gemäß Anspruch 1, **dadurch gekennzeichnet, dass** es sich um ein Diagnostikum handelt und die Diagnosevorrichtung im Kompartiment 3 lokalisiert ist.

4. Medizinisches Produkt gemäß Anspruch 1, **dadurch gekennzeichnet, dass** es sich um ein Kanülenfixierpflaster handelt und die Kanüle im Kompartiment 3 lokalisiert ist.

5. Medizinisches Produkt gemäß einem der Ansprüche 1 bis 4, **dadurch gekennzeichnet, dass** die Schicht 2 0 bis 5 % (w/w) eines Neutralöls, bezogen auf das trockene Gewicht dieser Schicht, als Verträglichmacher enthält.

6. Medizinisches Produkt gemäß einem der Ansprüche 1 bis 5, **dadurch gekennzeichnet, dass** die Schicht 2 0,5 bis 5 % (w/w) Dexpanthenol, bezogen auf das trockene Gewicht dieser Schicht, enthält.

7. Medizinisches Produkt gemäß einem der Ansprüche 1 bis 4, **dadurch gekennzeichnet, dass** die Schicht 2 nicht amin-resistente Polysiloxanpolymere umfasst, bei welchen nach der Polykondensation des Harz-Anteils und der Polydimethylsiloxanolgruppen die noch freien restlichen Silanolgruppen nicht mit Methylgruppen "gecapped" wurden.

8. Medizinisches Produkt gemäß einem der Ansprüche 1 bis 5 und 7, **dadurch gekennzeichnet, dass** die Schicht 2 0 bis 4 % (w/w) Silikonöl, bezogen auf das trockene Gewicht dieser Schicht, enthält.

9. Medizinisches Produkt gemäß einem der Ansprüche 1 bis 5, **dadurch gekennzeichnet, dass** die Schicht 2 Polyacrylate oder Vinyl-Acrylat-Copolymere umfasst, welche eine Säurezahl von kleiner 1 aufweisen.

10. Medizinisches Produkt gemäß einem der Ansprüche 1 bis 6, **dadurch gekennzeichnet, dass** die Schicht 2 Styren-Isopren-Styren-Block-Copolymere oder Styren-Butadien-Styren-Block-Copolymere in Kombination mit hydrierten Kohlenwasserstoffharzen und/oder Ölen wie Paraffinöl umfasst.

11. Verfahren zur Herstellung eines medizinisches Produkts gemäß einem der Ansprüche 1 bis 10, **dadurch gekennzeichnet, dass** man
- das Kompartiment 3 in an sich bekannter Weise herstellt und dessen hautseitige Klebefläche mit einer allseitig um mindestens 4 mm überstehenden wiederablösbaren Schutzschicht (6) abdeckt,
- den Hauttyp des Patienten beispielsweise nach W. E. Roberts klassifiziert und mit Hilfe dieser Klassifikation dann aus nicht amin-resistenten, stark wasserdampfdurchlässigen Polysiloxane, Polyacrylate ohne oder nur mit wenig freien Säuregruppen, Polyisobutylene, Polybutylene, Styren-Isopren-Styren-Block-Copolymeren oder Styren-Butadien-Styren-Block-Copolymeren oder deren Homo- und/oder Copolymeren umfassenden Gruppe diejenigen Polymere auswählt und gegebenenfalls aus den in den vorangehenden Ansprüchen beanspruchten Zusätzen diejenigen Zusätze auswählt, die erforderlich sind, damit das medizinische Produkt für eine Fixierdauer von mindesten 7 Tagen genutzt werden kann und dabei den Anforderungen an eine gute Hautverträglichkeit gerecht wird,
- daraus Beschichtungsmasse herstellt und diese auf einen Zwischenträger beschichtet und trocknet,
- die Rückschicht 1 auf das so erhaltene Laminat aus Schicht 2 und Zwischenträger auflaminiert und daraus ein Überpflaster einer solchen Größe und Form ausstanzt, welches das Kompartiment 3 allseitig um mindestens 4 mm überragt, und
- das so erhaltene Überpflaster schließlich nach Abziehen des Zwischenträgers auf die von der hautseitigen Klebefläche abgewandte Seite des Kompartiments 3 so aufklebt, dass sie dieses das Kompartiment allseitig um mindestens 4 mm überragt.

12. Kit-of-parts, umfassend
- ein auf der hautseitigen Klebefläche mit einer wiederablösbaren Schutzschicht (6) versehenes Kompartiment 3, das wie in einem der Ansprüche 1 bis 4 definiert ist, und
- mindestens ein auf einen Zwischenträger auflaminiertes wirkstofffreies Überpflaster aus
a) einer wasserdampfdurchlässigen Rückschicht(1) und
b) einer haftklebenden, nicht amin-resistente, stark wasserdampfdurchlässige Polysiloxane, Polyacrylate ohne oder nur mit wenig freien Säuregruppen, Polyisobutylene, Polybutylene, Styren-Isopren-Styren-Block-Copolymere oder Styren-Butadien-Styren-Block-Copolymere oder deren Homo- und/oder Copolymere umfassenden wirkstofffreien Polymerschicht (2).

13. Kit-of-parts gemäß Anspruch 12, umfassend
- mindestens zwei auf einen Zwischenträger auflaminierte, wie im Anspruch 12 definierte wirkstofffreien Überpflaster, die sich hinsichtlich der Zusammensetzung der Schicht 2, ihrer Größe und/oder ihrer Form unterscheiden.

14. Kit-of-parts, umfassend
- mindestens zwei auf einen Zwischenträger auflaminierte wirkstofffreie Überpflaster aus
a) einer wasserdampfdurchlässigen Rückschicht(1) und
b) einer haftklebenden, nicht amin-resistente, stark wasserdampfdurchlässige Polysiloxane, Polyacrylate ohne oder nur mit wenig freien Säuregruppen, Polyisobutylene, Polybutylene, Styren-Isopren-Styren-Block-Copolymere oder Styren-Butadien-Styren-Block-Copolymere oder deren Homo- und/oder Copolymere umfassenden wirkstofffreien Polymerschicht (2) wobei sich die Überpflaster hinsichtlich der Zusammensetzung der Schicht 2, ihrer Größe und/oder ihrer Form unterscheiden.

15. Kit-of-parts gemäß einem der Ansprüche 12 bis 14, in welchem die Schicht(en) 2 wie in einem der Ansprüche 5 bis 10 definiert ist/sind.

16. Verwendung eines Kit-of-parts gemäß einem der Ansprüche 12 bis 15 bei einem Verfahren zur Herstellung eines medizinisches Produkts, bestehend aus einem zentralen Kompartiment (3) und einem wirkstofffreien Überpflaster aus
a) einer wasserdampfdurchlässigen Rückschicht (1) und
b) einer haftklebenden, nicht amin-resistente, stark wasserdampfdurchlässige Polysiloxane, Polyacrylate ohne oder nur mit wenig freien Säuregruppen, Polyisobutylene, Polybutylene, Styren-Isopren-Styren-Block-Copolymere oder Styren-Butadien-Styren-Block-Copolymere oder deren Homo- und/oder Copolymere umfassenden wirkstofffreien Polymerschicht (2),
worin das Kompartiment 3 haftklebend ist und nach Abziehen der wiederablösbaren Schutzschicht (6) direkten Hautkontakt hat, bei welchem ein auf einen Zwischenträger auflaminiertes wirkstofffreies Überpflaster nach Abziehen des Zwischenträgers auf ein Kompartiment 3 aufgeklebt wird.
